# EUROPÄISCHE PATENTSCHRIFT

(11) **EP 2 331 699 B1**
(45) Veröffentlichungstag und Bekanntmachung des Hinweises auf die Patenterteilung: **27.01.2021**
(21) Anmeldenummer: 09744032.5
(22) Anmeldetag: 07.09.2009
(51) Int. Cl.: C12P 19/04, C12M 1/14

(54) **VERFAHREN ZUR HERSTELLUNG VON BAKTERIELL SYNTHETISIERTER CELLULOSE UND CELLULOSEHALTIGEM MATERIAL IN FLÄCHIGER FORM**
METHOD FOR THE PRODUCTION OF BACTERIALLY SYNTHESIZED CELLULOSE AND CELLULOSE-CONTAINING MATERIAL IN A PLANAR FORM
PROCÉDÉ DE PRODUCTION DE CELLULOSE ET DE MATIÈRE CELLULOSIQUE DE FORME PLATE SYNTHÉTISÉES PAR BACTÉRIES

(30) Priorität: 09.09.2008 DE 102008046644
(43) Veröffentlichungstag der Anmeldung: 15.06.2011
(73) Patentinhaber: JeNaCell GmbH, 07745 Jena (DE)
(72) Erfinder: KRALISCH, Dana, 07749 Jena (DE); HEßLER, Nadine, 96529 Mengersgereuth-Hämmern (DE); KLEMM, Dieter, 99425 Weimar (DE)
(74) Vertreter: Fuchs Patentanwälte Partnerschaft mbB
(86) Internationale Anmeldenummer: PCT/DE2009/001259
(87) Internationale Veröffentlichungsnummer: WO 2010/028632

(56) Entgegenhaltungen:
- WO-A2-2010/029044
- JP-A- 2005 082 704
- US-A- 6 071 727
- SAKAIRI N ET AL: "A method for direct harvest of bacterial cellulose filaments during continuous cultivation of Acetobacter xylinum" CARBOHYDRATE POLYMERS, APPLIED SCIENCE PUBLISHERS, LTD. BARKING, GB LNKD- DOI:10.1016/S0144-8617(97)00135-5, Bd. 35, Nr. 3-4, 4. März 1998 (1998-03-04) , Seiten 233-237, XP004126937 ISSN: 0144-8617 in der Anmeldung erwähnt
- JONAS R ET AL: "Production and application of microbial cellulose" POLYMER DEGRADATION AND STABILITY, BARKING, GB LNKD- DOI:10.1016/S0141-3910(97)00197-3, Bd. 59, Nr. 1-3, 3. Januar 1998 (1998-01-03), Seiten 101-106, XP025505631 ISSN: 0141-3910 [gefunden am 1998-01-03]
- TOKURA S ET AL: "Continuous harvest of cellulosic filament during cultivation of Acetobacter Xylinum", 10TH, CELLUCON CONFERENCE; CELLULOSIC PULPS, FIBRES AND MATERIALS; 1998; TURKU/ABO, FINLAND, WOODHEAD, CAMBRIDGE, GB, 1 January 2000 (2000-01-01), pages 3-12, XP008182544, DOI: 10.1533/9781845698546.4 ISBN: 978-1-85573-421-0 [retrieved on 2014-03-27]

## Beschreibung

Die Erfindung betrifft ein Verfahren zur Generierung von bakteriell synthetisierter Cellulose und cellulosehaltigem Material, um diese in flächiger Form homogen sowie mit variabel wählbarer Länge und Dicke herstellen zu können.

Konventionell wird bakteriell synthetisierte Cellulose (auch Bakteriencellulose, kurz: BC) in Standkultivierung mit einem Nährmedium gewonnen und das gebildete Material an der Grenzfläche zwischen Luft und Nährmedium abgeschöpft. Dabei ist jedoch die Größe des gewonnenen Vlieses von der Größe des Kultivierungsgefäßes abhängig und somit begrenzt.

Es sind auch bereits Lösungen bekannt, welche diese Abhängigkeit der Form des Bakteriencellulose-Materials vom Kultivierungsgefäß vermeiden sollen.

So wurden von Tokura et al. (S. Tokura, H. Tamura, M. Takai, T. Higuchi, H. Asano: Continuous harvest of cellulosic filament during cultivation of Acetobacter xylinum, Cellulosic Pulps, Fibres and Materials, International Cellucon Conference (on) Pulp for Papermaking: Fibre and Surface Properties and Other Aspects of Cellulose Technology, 10th, Turku/Abo, Finland, 2000, Meeting Date 1998), Tamura et al. (H. Tamura, Y. Tsuruta, S. Tokura: Shallow pan cultivation to enhance the yield of bacterial cellulose, Preprints of Symposia - American Chemical Society, Division of Fuel Chemistry, 1999, 44(2), 320-323), Sakairi et al. (N. Sakairi, H. Asano, M. Ogawa, N. Nishi, S. Tokura: A method for direct harvest of bacterial cellulose filaments during continuous cultivation of Acetobacter xylinum, Carbohydrate Polymers, 1998, 35(3-4), 233-237), Asano et al. (H. Asano, T. Kawai, H. Kawahara, N. Sakairi, N. Nishi, S. Tokura: Dependence of purification process on the property of bacterial cellulose obtained by direct filamentation from culture media, Kobunshi Ronbunshu, 1998, 55(4), 207-211) sowie Ogawa et al. (JP 10195713) jeweils eine Herstellung von BC-Fasern in einem flachen Becken unter Standkultivierung bei kontinuierlicher Aufwicklung der an der Oberfläche des Kulturmediums synthetisierten BC-Filamente beschrieben.

In diesem Kontext wurde auch über eine Steigerung der Biosynthese durch eine gleichzeitige Sauerstoffzufuhr berichtet (S. Tokura S., H. Tamura, M. Takai, T. Higuchi, H. Asano: Continuous harvest of cellulosic filament during cultivation of Acetobacter xylinum, Cellulosic Pulps, Fibres and Materials, International Cellucon Conference (on) Pulp for Papermaking: Fibre and Surface Properties and Other Aspects of Cellulose Technology, 10th, Turku/Abo, Finland, 2000, Meeting Date 1998).

Bei einem solchen Verfahren entsteht jedoch keine Bakteriencellulose in flächiger Form, sondern es werden lediglich zusammengelagerte Filamente mit uneinheitlicher innerer Struktur generiert.

Es ist auch eine Herstellung von Bakteriencellulose durch einen linearen Bioreaktor bekannt, der mit einem Förderband versehen ist und kontinuierlich durch das vorliegende Nährmedium geleitet wird, oder durch einen Rotary-Disk-Reactor (US 6071727).

In beiden Fällen werden partiell getrocknete BC-Folien aus einem BC-Gelfilm erhalten. Bei diesem Ansatz entsteht jedoch ein sehr inhomogenes Material ohne definierte Ausmaße (Höhe, Länge, Breite).

Bekannt ist auch, Biofilme aus Bakteriencellulose unter Spannung zu dünnen Folien aufzuwickeln.

Nach Tokura und Tamura (JP 2005082704) führt die Aufwicklung von an der Grenzfläche zwischen Luft und Nährmedium kultivierten BC-Biofilmen zu flächiger BC. Hierbei wird jedoch die BC einer mechanischen Kraft ausgesetzt und ihre Struktur verändert. Zudem erfolgt die Aggregation des Biofilms zu dünnen Folien nicht während der Biosynthese im Reaktor, sondern durch eine vorgegebene Anordnung der synthetisierten Schichten während des Aufwicklungsprozesses. Somit lassen sich auf diese Weise keine Vliese mit homogener 3-dimensionaler Netzwerkstruktur erzielen.

Die Standkultivierung hat darüber hinaus den Nachteil, dass sie sehr flächenintensiv und somit für eine großtechnische Produktion nicht geeignet ist. Zudem kann auf diesem Wege keine gleich bleibende, optimale Versorgung an Nährmedium gewährleistet werden. Die Effizienz derartiger Verfahren ist somit geringer als bei kontinuierlicher Versorgung an Nährmedium bei optimaler Verfügbarkeit der Nährmediumsinhaltsstoffe.

Vor diesem Hintergrund sind erste Ansätze einer semi-kontinuierlichen Darstellung bekannt.

So ist eine Zunahme der BC-Ausbeuten durch eine kontinuierliche Zuführung von Nährmedium unterhalb des BC-Vlieses während der Biosynthese unter statischen Kultivierungsbedingungen bekannt (JP 06253877). Das gewonnene Vlies entspricht jedoch der Reaktorgeometrie. Andere Vliesabmessungen sind damit nicht möglich.

Bekannt ist auch eine kontinuierliche Abtrennung der synthetisierten Bakteriencellulose im Rahmen eines Fermentationsprozesses, wodurch die Konzentration im Reaktor gering gehalten wird (JP 08033495).

Vorgestellt wurde auch bereits eine konstante Isolierung von Bakteriencellulose aus dem Kulturmedium eines Fermenters mittels Floating separation und Abtrennung über einem Filter (JP 08033494).

In JP 11018758 wird ein Spinner culture tank mit unterschiedlich eingelagerten Laufrädern offenbart, wodurch zusätzlich eine gute Versorgung mit Sauerstoff gewährleistet war. In keiner der vorgeschlagenen Varianten entsteht jedoch ein homogenes, flächiges Produkt.

In WO 2004050986 wird eine Methode vorgestellt, bei welcher das Nährmedium mittels eines mechanischen Mixers hergestellt, in Kästen gegeben und mit recycelter oder nicht-recycelter Vorkultur beimpft wird. Die Temperatur der Anlage wird durch eine Ummantelung konstant gehalten. Die Apparatur dient der Herstellung von Bakteriencellulose-Folien.

Dieses Verfahren wurde von Farah et al. weiterentwickelt. Dabei ist es nicht mehr erforderlich, das Kulturmedium in den Kästen zu erneuern. Das erhaltene Bakteriencellulose-Vlies wird über dem entsprechenden Kasten entwässert und die Kultivierung fortgesetzt. Dies kann so lange durchgeführt werden, bis das Volumen nur noch 15-25 % vom Ausgangsvolumen aufweist. Danach muss neues Nährmedium zugeführt werden (WO 2006066377).

Der Nachteil bleibt jedoch bestehen, dass die gewonnene Fläche an bakteriell synthetisierter Cellulose von der Form der verwendeten Reaktionsgefäße abhängt und somit einerseits in der Größe limitiert und anderseits nicht variabel ist. Darüber hinaus schwankt die Nährmediumskonzentration im Verfahrensverlauf. Dadurch ist keine gleich bleibende, optimale Nährmittelverfügbarkeit gegeben.

Ferner ist ein kontinuierlicher Herstellungsprozess auf Basis eines Aerosolverfahrens bekannt (DE 100 22 751). Dabei werden die Nährstoffe nicht wie bei anderen Verfahren von unten den Bakterien zugeführt, sondern durch feines Versprühen von oben. So ist es möglich, Bakteriencellulose-Vliese in großer Dicke zu produzieren. Allerdings ist auch bei dieser Methode die Größe des gewonnenen Vlieses von der Größe des Kultivierungsgefäßes abhängig. Außerdem besitzt das Vlies keine homogenen Materialeigenschaften.

Der Erfindung liegt die Aufgabe zu Grunde, ein universell anwendbares und für eine großtechnische Produktion geeignetes Verfahren zu schaffen, welches eine effiziente Herstellung homogener, flächiger bakteriell synthetisierter Cellulose sowie cellulosehaltiger Materialien in einer von der Reaktorgeometrie unabhängigen sowie beliebig wählbaren definierten Länge und Dicke ermöglicht.

Die Erfindung bezieht sich auf ein Verfahren zur Herstellung von bakteriell synthetisierter Cellulose und cellulosehaltigem Material in flächiger Form, welche in einem Kulturgefäß an der Grenzfläche eines darin befindlichen und mit einer Bakterienkultur beimpften Nährmediums zur Luft kultiviert, anschließend entnommen und nach Entnahme aus dem Kulturgefäß der Einwirkung eines Bakterien tötenden Behandlungsmediums ausgesetzt wird, dadurch gekennzeichnet,
- dass jeweils der an der Grenzfläche des mit der Bakterienkultur beimpften Nährmediums zur Luft in der definierten Dicke fertig kultivierte Teil der bakteriell synthetisierten Cellulose bzw. des bakteriell synthetisierten cellulosehaltigen Materials ohne Abtrennung von dem noch nicht fertig kultivierten Teil der Cellulose bzw. des cellulosehaltigen Materials als zusammenhängendes flächiges vlies- oder folienartiges Band in Ebene der Grenzfläche des mit der Bakterienkultur beimpften Nährmediums aus einem wannenartigen Kulturgefäß herausgezogen wird, wobei der von diesem Band noch nicht fertig kultivierte Teil der Cellulose bzw. des cellulosehaltigen Materials jeweils noch im Kulturgefäß verbleibt, dort ungestört von der Entnahme des Teils des Bandes mit der fertig kultivierten Cellulose bzw. dem cellulosehaltigen Material weiterkultiviert wird und nach Erreichen der definierten Dicke, unabgetrennt von dem bereits entnommenen Teil des Bandes fertig kultivierter Cellulose bzw. fertig kultivierten cellulosehaltigen Materials sowie unabgetrennt von einem weiteren im Kulturgefäß verbleibenden Teil des Bandes noch nicht fertig kultivierter Cellulose bzw. noch nicht fertig kultivierten cellulosehaltigen Materials, in demselben Band weiter aus dem Kulturgefäß entnommen wird,
- dass jeweils der Teil des Bandes mit der fertig kultivierten bakteriell synthetisierten Cellulose bzw. mit dem fertig kultivierten bakteriell synthetisierten cellulosehaltigen Material nach der Entnahme aus dem Kulturgefäß jeweils dem Bakterien tötenden Behandlungsmedium ausgesetzt wird, durch welches ein weiterer Kultivierungsprozess der Cellulose bzw. des cellulosehaltigen Materials dieses Teils des Bandes verhindert wird,
- dass der aus dem Kulturgefäß entnommene Teil des Bandes mit der jeweils fertig kultivierten bakteriell synthetisierten Cellulose bzw. des fertig kultivierten bakteriell synthetisierten cellulosehaltigen Materials nach der Einwirkung des Bakterien tötenden Behandlungsmediums in der gewünschten Länge des flächigen vlies- oder folienartigen Materials vom übrigen Band abgetrennt wird, wobei das Band mit dem jeweils fertig kultivierten Teil der bakteriell synthetisierten Cellulose bzw. des fertig kultivierten bakteriell synthetisierten cellulosehaltigen Materials kontinuierlich oder schrittweise aus dem Kulturgefäß entnommen wird und in einem nicht zur Verwertung der Cellulose bzw. des cellulosehaltigen Materials vorgesehenen Anfangsbereich des Bandes mit einer im Wesentlichen oben und/oder unten an das Band greifenden Entnahmevorrichtung in Kontakt gebracht wird. Dazu wird die bakteriell synthetisierte Cellulose bzw. das bakteriell synthetisierten cellulosehaltigen Material in einem wannenartigen Gefäß (Reaktor) kultiviert, dessen Breite vorzugsweise der Breitenabmessung des zu gewinnenden vlies- oder folienartigen "Bandes" entspricht, so dass dieses in der Breite nicht zwingend zurechtgeschnitten werden muss. Der jeweils fertig kultivierte Teil der bakteriell synthetisierten Cellulose bzw. des bakteriell synthetisierten cellulosehaltigen Materials wird durch Zug und/oder Hub vom Anfang des besagten Bandes dieser Cellulose bzw. des cellulosehaltigen Materials her aus dem Kulturgefäß (Reaktor) entnommen, wobei der noch nicht fertig kultivierte Teil der Cellulose bzw. des cellulosehaltigen Materials als Bestandteil des besagten "Bandes" jeweils im Kulturgefäß durch die Entnahme des zusammenhängenden "Bandes" weitertransportiert wird, aber noch im Kulturgefäß verbleibt und dort ungestört von der Entnahme weiterkultiviert wird bis auch dieser Teil im Kultivierungsprozess seine vorbestimmte Schichtdicke der Cellulose bzw. des cellulosehaltigen Materials erreicht hat.

Dabei bildet sich am Bandende (Anfang des Reaktors) durch den Kultivierungsprozess stets neue Cellulose bzw. cellulosehaltiges Material aus, wodurch sich das "Band" fortwährend und nahtlos verlängert.

Dann wird das "Band" mit diesem nunmehr zwischenzeitlich fertig kultivierten Teil der Cellulose bzw. des cellulosehaltigen Materials unabgetrennt vom übrigen Bandteil weiter entnommen, wobei der noch nicht fertig kultivierte Teil des "Bandes" wiederum im Kulturgefäß zur Fortsetzung des Kultivierungsprozesses verbleibt, solange die gewünschte Bandlänge der zu gewinnenden Cellulose bzw. des cellulosehaltigen Materials noch nicht erreicht ist.

Auf diese Weise wird aus dem Kulturgefäß schrittweise oder kontinuierlich ein konsistentes "Band" mit jeweils flächiger fertig kultivierter Cellulose bzw. cellulosehaltigen Materials entnommen, dessen Länge weitaus größer als die Länge des Kulturgefäßes sein kann und auch von dessen Gefäßlänge in keiner Weise begrenzt wird. Auch die Dicke der Cellulose bzw. des cellulosehaltigen Materials wird von der Geometrie des Kulturgefäßes weitgehend nicht begrenzt.

Für den weiteren Kultivierungsprozess ist lediglich dafür zu sorgen, dass die Kultivierungsbedingungen erhalten bleiben. Entweder ist für den Gesamtprozess der Kultivierung der gesamten (gewünschten) Bandlänge hinreichend Nährmedium und Sauerstoff vorhanden oder es werden während des Kultivierungsprozesses kontinuierlich oder diskontinuierlich neues Nährmedium bzw. dessen verbrauchte Komponenten und/oder neue Luft zugeführt bzw. ausgetauscht.

Der Teil des "Bandes" mit der jeweils fertig kultivierten bakteriell synthetisierten Cellulose bzw. mit dem fertig kultivierten bakteriell synthetisierten cellulosehaltigen Material wird ungeachtet des im Kulturgefäß zur Weiterkultivierung verbleibenden Bandteil jeweils nach der Entnahme aus dem Kulturgefäß einem Bakterien tötenden Behandlungsmedium (z. B. eine hochtemperierte Flüssigkeit oder gasförmiges Medium, wie Wasserdampf) ausgesetzt, durch welches ein weiterer Kultivierungsprozess der Cellulose bzw. des cellulosehaltigen Materials dieses Teils des "Bandes" unterbunden wird. Dies kann beispielsweise durch Besprühen erfolgen, sofern dabei das Behandlungsmedium nicht in das Kulturgefäß gelangen kann, oder das "Band" wird nach der Entnahme aus dem Kulturgefäß in das Behandlungsmedium getaucht (z. B. unter Ziehen durch eine dafür vorgesehene Wanne).

Ist das "Band" mit der zu gewinnenden flächigen bakteriell synthetisierten Cellulose bzw. mit dem bakteriell synthetisierten cellulosehaltigen Material in der (unabhängig von der Länge des Kulturgefäßes) gewünschten Länge insgesamt fertig kultiviert, wird das "Band" vorzugsweise nach der Einwirkung des Bakterien tötenden Behandlungsmediums in der gewünschten Länge des flächigen vlies-oder folienartigen "Bandes" vom übrigen Band abgetrennt und somit in der Länge portioniert, welche, wie bereits beschrieben, weitaus größer als das Kulturgefäß sein kann, in welchem die Cellulose bzw. das cellulosehaltige Material an der Grenzfläche des mit der Bakterienkultur beimpften Nährmediums zur Luft homogener Struktur in der vorgegebenen Bandbreite kultiviert.

Durch den erfindungsgemäßen Herstellungsprozess wirken auf die Cellulose bzw. das cellulosehaltige Material keine äußeren Bedingungen, beispielsweise Druckeinwirkungen, welche das Wachstum und den Erhalt der generierten flächigen Cellulose bzw. des cellulosehaltigen Materials beeinträchtigen, so dass die natürlich gewachsene, homogene Cellulosestruktur auch bei sehr großen Längen des besagten "Bandes" der Cellulose bzw. des cellulosehaltigen Materials erhalten bleibt. Mit unterschiedlichen Geometrien der Kulturgefäße bzw. mit einstellbaren Breiten der Kultivierungszone des Kulturgefäßes sind auch in großen Grenzen beliebig wählbare Bandbreiten möglich.

Weiterhin ist von Vorteil, dass durch das vorgeschlagene Verfahren bei der Entnahme aus dem Kulturgefäß das vom Stand der Technik semikontinuierlicher bis kontinuierlicher BC-Darstellung bekannte nachteilige Zusammenwachsen verschiedener, aufeinander gelagerter Schichten und somit die Bildung eines inhomogenen BC-Materials mit uneinheitlichen Materialeigenschaften ausgeschlossen wird. Mit Hilfe des vorgeschlagenen Verfahrens kann flächige Bakteriencellulose bzw. cellulosehaltiges Material dagegen in einer sehr hohen und vor allem reproduzierbaren Qualität gewonnen werden, die bisher nur über eine Standkultivierung erzielt werden konnte, die jedoch mit den eingangs benannten Nachteilen behaftet ist.

Mit der Erfindung kann technologisch vorteilhaft und wirtschaftlich effizient sowie auch für großtechnische Produktionen geeignet, flächige vlies- oder folienartige Cellulose bzw. cellulosehaltiges Material mit beliebig wählbaren Abmessungen und dennoch homogener Materialstruktur generiert werden.

Vorzugsweise nach oder auch vor der Portionierung des "Bandes" wird die fertig kultivierte und dem Behandlungsmedium zwecks besagter Abtötung der Bakterien ausgesetzte Cellulose bzw. das cellulosehaltige Material gereinigt (wiederum beispielsweise ein oder mehrere Sprüh-und/oder Tauchvorgänge).

In den Unteransprüchen sind vorteilhafte Ausgestaltungsmerkmale des Verfahrens, beispielsweise Möglichkeiten zur Entnahme des Bandes aus dem Kulturgefäß, aufgeführt.

So kann auch sehr vorteilhaft mit der Entnahmegeschwindigkeit des "Bandes" aus dem Kulturgefäß die Dicke der kultivierten Cellulose bzw. des cellulosehaltigen Materials beeinflusst und gesteuert werden.

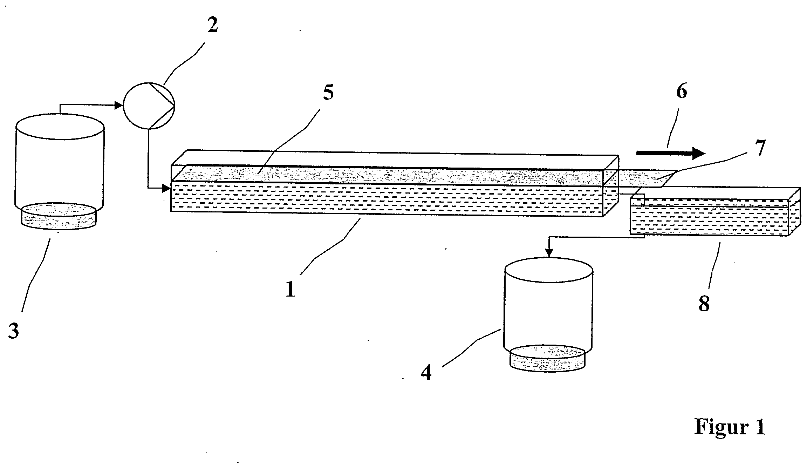

Die Erfindung soll nachstehend anhand eines in der Zeichnung dargestellten Ausführungsbeispiels näher erläutert werden.

Die Figur zeigt in schematischer Darstellung den Ablauf des Kultivierungsverfahrens zur Gewinnung bakteriell synthetisierter Cellulose in flächiger Form mit variabel wählbarer Länge und Dicke.

Zunächst wird in an sich bekannter Weise eine Bakterienkulturlösung mit dem cellulosebildenden Bakterium vom Stamm *Gluconacetobacter xylinus* vorkultiviert, so dass diese in ausreichender Menge zur Verfügung steht (100 - 2000 ml, vorzugsweise 400 - 600 ml, Bakterienkulturlösung, bezogen auf 10 l zu beimpfendes Nährmedium).

Des Weiteren erfolgen (ebenfalls auf bekannte Art) die Herstellung und Sterilisation eines Nährmediums nach Hestrin-Schramm (20 g D-(+)-Glucose, 5 g Bactopepton, 5 g Hefeextrakt, 3,4 g Dinatriumhydrogenphosphat Dihydrat, 1,15 g Citronensäure Monohydrat pro Liter Nährmedium, aufgefüllt mit entionisiertem Wasser) sowie die Sterilisation aller benötigten Gefäße und des Zubehörs (aus Übersichtsgründen nicht in der Zeichnung dargestellt).

Ist dies geschehen, wird ein wannenartiges Kulturgefäß 1 als Reaktor aus korrosionsfreiem, chemikalien- und hitzebeständigem Material über eine Pumpe 2 an einen Vorratsbehälter 3 zur späteren Zufuhr von frischem Nährmedium angeschlossen sowie mit einem Auffangbehälter 4 zum Auffangen von verbrauchtem Nährmedium angeschlossen.

In der Zeichnung aus Übersichtsgründen ebenfalls nicht dargestellte elektrische Einheiten und Geräte zur Gewährleistung geeigneter Kultivierungsbedingungen werden in Betrieb genommen. Insbesondere werden eine Kultivierungstemperatur von 20 °C - 32 °C, vorzugsweise 28 °C - 30 °C und eine Luftzufuhr von 1 - 200 l/min eingestellt.

Nach Einstellung der besagten Reaktions- und Kultivierungsbedingungen wird das Kulturgefäß 1 mit der vorkultivierten Bakterienkulturlösung und dem Nährmedium befüllt. Mit dieser Beimpfung des Nährmediums durch die Bakterien des *Gluconacetobacter Stammes* beginnt die Biosynthese der Bakteriencellulose und es bildet sich an der Grenzfläche dieser hergestellten Kulturlösung (mit der Bakterienkultur beimpftes Nährmedium) eine flächige Schicht bakteriell synthetisierter Cellulose (Bakteriencellulose 5), die während ihres Kultivierungsprozesses in ihren Abmessungen zunächst durch die Oberflächengeometrie des Kulturgefäßes 1 begrenzt wird.

Über die Pumpe 2 erfolgt kontinuierlich oder bei Bedarf eine Zufuhr von frischem Nährmedium aus dem dafür vorgesehenen Vorratsbehälter 3 in das Kulturgefäß 1. Entsprechend dieser Zufuhr von neuerem Nährmedium an einer Stirnseite des Kulturgefäßes 1 wird an dessen gegenüberliegenden anderen Stirnseite durch den Kultivierungsprozess verbrauchte Nährlösung in den Auffangbehälter 4 abgeführt. So wird während des gesamten Kultivierungsprozesses eine optimale Versorgung der im Kulturgefäßes 1 befindlichen Bakterienkultur mit allen erforderlichen Nährstoffen gewährleistet. Zu- und Ablauf von neuem und verbrachtem Nährmedium sind im Kulturgefäß 1 an den besagten gegenüberliegenden Stirnseiten jeweils unterhalb der Grenzfläche des mit der Bakterienkultur beimpftem Nährmediums, an welcher die Bakteriencellulose kultiviert wird, angeordnet. Die Position des Ablaufs bestimmt dabei die Füllstandshöhe des mit der Bakterienkultur beimpftem Nährmediums im Kulturgefäß 1.

Neben der genannten kontinuierlichen Versorgung der Bakterienkultur mit den notwendigen Nährstoffen gewährleistet die Zufuhrgeschwindigkeit von neuem Nährmedium auch die Einhaltung der für die Austragung der flächigen Bakteriencellulose 5 erforderlichen Füllstandshöhe. Die Zufuhrgeschwindigkeit liegt bei 0,5 - 20 ml/min.

Nach einer Kultivierungsdauer von 10 - 16 Tagen wird flächige Bakteriencellulose 5 mit einer Dicke von 0,5 - 1,6 cm erhalten, deren Maße der Reaktorgeometrie des Kulturgefäßes 1 in horizontaler Sicht entsprechen.

Ist die gewünschte Dicke der an der Grenzfläche des mit der Bakterienkultur beimpften Nährmediums zur Luft kultivierten Bakteriencellulose 5 im Kulturgefäß 1 erreicht, erfolgt ein stufenweiser oder kontinuierlicher Austrag dieser flächigen Bakteriencellulose 5 über ein aus Übersichtsgründen nicht dargestelltes Transportsystem am Reaktorausgang (rechte Seite des in der Figur gezeigten Kulturgefäßes 1). Auf diese Weise wird die fertig kultivierte Bakteriencellulose als flächiges, zusammenhängendes "Band" in Ebene der Grenzfläche der Kulturlösung (mit der Bakterienkultur beimpftes Nährmedium) vom Kulturgefäß 1 (ggf. unter leichtem Anheben) aus diesem herausgezogen. Die Entnahme an sich sowie die Transportrichtung dieses "Bandes" werden in der Zeichnung durch einen Pfeil 6 symbolisiert. Für diese Entnahme tastet die nicht dargestellte Transporteinrichtung, beispielsweise mittels Klemmen, Pressen oder durch Einrasten von Mitnameelementen, wie Widerhaken, an einen nicht zur Verwertung der Bakteriencellulose 5 vorgesehenen Anfangsbereich 7 des "Bandes" form- und kraftschlüssig an. Es könnten auch Antastelemente in diesen nicht zur Verwertung der Bakteriencellulose 5 vorgesehenen Anfangsbereich 7 einkultiviert worden sein, an welche die Transporteinrichtung, beispielsweise durch Rast- oder Greifelemente bzw. durch kletthalterungs- bzw. snap-in- artige oder andere Elemente angreift.

Durch diese Entnahme wird jeweils der Teil des "Bandes" mit der im Kulturgefäß 1 fertig kultivierten Bakteriencellulose 5 aus dem Kulturgefäß 1 herausgezogen. Der Teil des "Bandes" mit der noch nicht fertig kultivierten Bakteriencellulose 5 wird zwar durch den Transport des zusammenhängenden "Cellulosebandes" im Kulturgefäß 1 in Richtung Reaktorausgang des Kulturgefäßes 1 bewegt, verbleibt aber ungeachtet von der Entnahme des Bandteils mit der bereits fertig kultivierten Bakteriencellulose 5 im Kulturgefäß 1 und kultiviert dort unter unveränderten Reaktionsbedingungen weiter, bis auch dieser Bandteil aus fertig kultivierter Bakteriencellulose 5 besteht und eine gewünschte Vlies- oder Foliendicke aufweist sowie das "Band" weiter aus dem Kulturgefäß 1 entnommen wird.

Die Variation der Dicke des gewonnenen BC-Vlieses (oder je nach Dicke der BC-Folie) erfolgt anhand der Transportgeschwindigkeit, mit welcher das "Band" aus dem Kulturgefäß 1 entnommen wird.

Unmittelbar nach Austrag des jeweiligen Bandteils mit der fertig kultivierten Bakteriencellulose 5 aus dem Kulturgefäß 1 wird der betreffende Bandteil durch ein Bakterien abtötendes Behandlungsmedium, welches sich in einem wannenförmigen Behandlungsgefäß 8 unmittelbar am Reaktorausgang des Kulturgefäßes 1 befindet, ausgesetzt. Als Behandlungsmedium kann vorzugsweise Wasser mit einer Temperatur von über 60 °C Verwendung finden. Für diese Behandlung wird das aus dem Kulturgefäß 1 entnommene "Band" unmittelbar nach der Entnahme von der nicht in der Zeichnung dargestellten Transporteinrichtung mit entsprechenden Transportelementen durch das Behandlungsgefäß 8 gezogen, in welchem das "Band" in das Bakterien abtötende Behandlungsmedium eintaucht. Alternativ könnte der aus dem Kulturgefäß 1 jeweils entnommene Bandteil auch mit dem Behandlungsmedium besprüht werden, wobei allerdings gewährleistet sein muss, dass das versprühte Behandlungsmedium nicht rückwärtig in das Kulturgefäß 1 gelangt und dort das Kulturmedium zur Kultivierung der Bakteriencellulose 5 beeinträchtigt.

Zusätzlich kann zur Entfernung der bakteriellen Rückstände sowie von Nährmediumsbestandteilen eine Reinigung der flächigen Bakteriencellulose 5, vorzugsweise durch Kochen in einer leicht alkalischen Reinigungslösung (0,1 N) erfolgen (in der Zeichnung nicht dargestellt).

Der Entnahmeprozess des "Bandes", bei dem jeweils der Bandteil mit der fertig kultivierten Bakteriencellulose 5 aus dem Kulturgefäß 1 ausgetragen wird und der Bandteil mit der noch nicht fertig kultivierten Bakteriencellulose 5 jeweils im Kulturgefäß 1 verbleibt, wobei sich im Reaktoranfangsbereich (in Reaktordarstellung links) des Kulturgefäßes 1 stets neue Bakteriencellulose 5 bildet und sich dadurch das "Band" fortwährend nahtlos "verlängert", wird solange fortgeführt, bis eine Bandlänge mit der gewünschten Länge der verwertbaren flächigen vlies-oder folienartigen Bakteriencellulose erreicht ist. In diesem Fall, wird das Band (vorzugsweise nach Behandlung mit der Bakterien abtötenden Behandlungsflüssigkeit und ggf. nach erfolgter Reinigung) unter Trennung des "Bandes" vom übrigen Band frei wählbar portioniert.

Die Erfindung ist nicht auf die Synthese reiner Bakteriencellulose beschränkt. Mit demselben beschriebenen Verfahrensablauf können auch bakteriencellulosehaltige Materialien in flächiger Form mit variabel wählbarer Länge und Dicke hergestellt werden, wobei zur Komposit-Bildung lediglich während und/oder nach der Biosynthese entsprechende geeignete Kompositbildner zugeführt werden.

### Bezugszeichenliste

- 1: - Kulturgefäß (Reaktor)
- 2: - Pumpe (zur Zuführung neuer Nährlösung aus dem Vorratsbehälter 3)
- 3: - Vorratsbehälter (für neues Nährmedium)
- 4: - Auffangbehälter (für verbrauchtes Nährmedium)
- 5: - Bakteriencellulose
- 6: - Pfeil (Transportrichtung der Entnahme)
- 7: - Anfangsbereich des "Bandes"
- 8: - Behandlungsgefäß (zum Abtöten von Bakterien)

## Patentansprüche

1. Verfahren zur Herstellung von bakteriell synthetisierter Cellulose und cellulosehaltigem Material in flächiger Form, welche in einem Kulturgefäß an der Grenzfläche eines darin befindlichen und mit einer Bakterienkultur beimpften Nährmediums zur Luft kultiviert, anschließend entnommen und nach Entnahme aus dem Kulturgefäß der Einwirkung eines Bakterien tötenden Behandlungsmediums ausgesetzt wird, **dadurch gekennzeichnet,**
• **dass** jeweils der an der Grenzfläche des mit der Bakterienkultur beimpften Nährmediums zur Luft in der definierten Dicke fertig kultivierte Teil der bakteriell synthetisierten Cellulose bzw. des bakteriell synthetisierten cellulosehaltigen Materials ohne Abtrennung von dem noch nicht fertig kultivierten Teil der Cellulose bzw. des cellulosehaltigen Materials als zusammenhängendes flächiges vlies- oder folienartiges Band in Ebene der Grenzfläche des mit der Bakterienkultur beimpften Nährmediums aus einem wannenartigen Kulturgefäß herausgezogen wird, wobei der von diesem Band noch nicht fertig kultivierte Teil der Cellulose bzw. des cellulosehaltigen Materials jeweils noch im Kulturgefäß verbleibt, dort ungestört von der Entnahme des Teils des Bandes mit der fertig kultivierten Cellulose bzw. dem cellulosehaltigen Material weiterkultiviert wird und nach Erreichen der definierten Dicke, unabgetrennt von dem bereits entnommenen Teil des Bandes fertig kultivierter Cellulose bzw. fertig kultivierten cellulosehaltigen Materials sowie unabgetrennt von einem weiteren im Kulturgefäß verbleibenden Teil des Bandes noch nicht fertig kultivierter Cellulose bzw. noch nicht fertig kultivierten cellulosehaltigen Materials, in demselben Band weiter aus dem Kulturgefäß entnommen wird,
• **dass** jeweils der Teil des Bandes mit der fertig kultivierten bakteriell synthetisierten Cellulose bzw. mit dem fertig kultivierten bakteriell synthetisierten cellulosehaltigen Material nach der Entnahme aus dem Kulturgefäß jeweils dem Bakterien tötenden Behandlungsmedium ausgesetzt wird, durch welches ein weiterer Kultivierungsprozess der Cellulose bzw. des cellulosehaltigen Materials dieses Teils des Bandes verhindert wird,
• **dass** der aus dem Kulturgefäß entnommene Teil des Bandes mit der jeweils fertig kultivierten bakteriell synthetisierten Cellulose bzw. des fertig kultivierten bakteriell synthetisierten cellulosehaltigen Materials nach der Einwirkung des Bakterien tötenden Behandlungsmediums in der gewünschten Länge des flächigen vlies- oder folienartigen Materials vom übrigen Band abgetrennt wird, wobei das Band mit dem jeweils fertig kultivierten Teil der bakteriell synthetisierten Cellulose bzw. des fertig kultivierten bakteriell synthetisierten cellulosehaltigen Materials kontinuierlich oder schrittweise aus dem Kulturgefäß entnommen wird und in einem nicht zur Verwertung der Cellulose bzw. des cellulosehaltigen Materials vorgesehenen Anfangsbereich des Bandes mit einer im Wesentlichen oben und/oder unten an das Band greifenden Entnahmevorrichtung in Kontakt gebracht wird.

2. Verfahren gemäß Anspruch 1, **dadurch gekennzeichnet, dass** im Anfangsbereich des Bandes ein- oder mehrere Mitnahmeelemente in die Bakteriencellulose bzw. in das Bakteriencellulosehaltige Material einkultiviert werden.

3. Verfahren gemäß Anspruch 2, **dadurch gekennzeichnet, dass** an die Mitnahmeelemente nach dem Prinzip der Kletthalterung durch eine Entnahmevorrichtung angetastet werden kann.

4. Verfahren gemäß Anspruch 1, **dadurch gekennzeichnet, dass** durch die Geschwindigkeit, mit welcher das Band aus dem Kulturgefäß entnommen wird, die Dicke des Teils des Bandes im Kulturgefäß mit der jeweils fertig kultivierten bakteriell synthetisierten Cellulose bzw. mit dem fertig kultivierten bakteriell synthetisierten cellulosehaltigen Material bestimmt wird.

5. Verfahren nach Anspruch 1, **dadurch gekennzeichnet, dass** das Band mit dem jeweils fertig kultivierten Teil der bakteriell synthetisierten Cellulose bzw. des bakteriell synthetisierten cellulosehaltigen Materials zwecks Behandlung mit dem Bakterien tötenden Behandlungsmedium nach Entnahme aus dem Kulturgefäß durch einen vorzugsweise wannenartigen Behälter geführt wird, in welchem sich das Behandlungsmedium befindet.

6. Verfahren nach Anspruch 1, **dadurch gekennzeichnet, dass** das Band mit dem jeweils fertig kultivierten Teil der bakteriell synthetisierten Cellulose bzw. des bakteriell synthetisierten cellulosehaltigen Materials nach der Entnahme aus dem Kulturgefäß und nach der Einwirkung eines Bakterien tötenden Behandlungsmediums einer Reinigungsbehandlung ausgesetzt wird.

7. Verfahren nach Anspruch 6, **dadurch gekennzeichnet, dass** die Reinigungsbehandlung durch ein- oder mehrmaliges Besprühen des Bandes mit einer Reinigungsflüssigkeit erfolgt.

8. Verfahren nach Anspruch 6, **dadurch gekennzeichnet, dass** die Reinigungsbehandlung durch Eintauchen des Bandes in eine Reinigungsflüssigkeit erfolgt.

9. Verfahren nach Anspruch 1, **dadurch gekennzeichnet, dass** während der Kultivierung der bakteriell synthetisierten Cellulose bzw. des bakteriell synthetisierten cellulosehaltigen Materials im Kulturgefäß zum Zweck konstant bleibender Bedingungen des Kultivierungsprozesses jeweils verbrauchte Komponenten des mit der Bakterienkultur beimpften Nährmediums gegen entsprechend neue Komponenten ausgetauscht werden.

10. Verfahren nach Anspruch 1, **dadurch gekennzeichnet, dass** während der Kultivierung der bakteriell synthetisierten Cellulose bzw. des bakteriell synthetisierten cellulosehaltigen Materials im Kulturgefäß die Temperatur des Nährmediums und/oder der Luft in Abhängigkeit der kultivierenden Bakteriencellulose und/oder der für die Impfung des Nährmediums verwendeten Bakterienkultur in einem Bereich zwischen 4 °C und 60 °C eingestellt wird.

11. Verfahren nach Anspruch 1, **dadurch gekennzeichnet, dass** als Bakterienkultur zur Impfung des Nährmediums ein Cellulose-produzierender Bakterienstamm Verwendung findet.

## Claims

1. A method for the production of bacterially synthesized cellulose and cellulose-containing material in a planar form which is cultured in a culture vessel at the interface of a nutritional medium being present therein and being inoculated with a bacterial culture with air, is subsequently removed and after the removal from the culture vessel is subjected to the exposure of a bactericidal treatment medium, **characterized in**
• **that** the part of the bacterially synthesized cellulose and the bacterially synthesized cellulose-containing material, respectively, each being completely cultured in the defined thickness at the interface of the nutritional medium being inoculated with the bacterial culture with air is pulled out of a trough-like culture vessel as a coherent planar fleece- or film-like ribbon in the plane of the interface of the nutritional medium being inoculated with the bacterial culture without detachment of the still not completely cultured part of the cellulose and the cellulose-containing material, respectively, wherein the part of the ribbon of the cellulose and the cellulose-containing material, respectively, which is still not completely cultured each still remains in the culture vessel, is further cultured there undisturbed by the removal of the part of the ribbon with the completely cultured cellulose and the cellulose-containing material, respectively, and is further removed from the culture vessel after achieving the defined thickness in the same ribbon, not detached from the already removed part of the ribbon of completely cultured cellulose and completely cultured cellulose-containing material, respectively, as well as not detached from a further part of the ribbon of still not completely cultured cellulose and still not completely cultured cellulose-containing material, respectively, which remains in the culture vessel,
• **that** the part of the ribbon with the completely cultured bacterially synthesized cellulose and with the completely cultured bacterially synthesized cellulose-containing material, respectively, each after the removal from the culture vessel is respectively subjected to the bactericidal treatment medium through which a further culturing process of the cellulose and the cellulose-containing material, respectively, of this part of the ribbon is prevented,
• **that** the part of the ribbon with the respectively completely cultured bacterially synthesized cellulose and the completely cultured bacterially synthesized cellulose-containing material, respectively, being removed from the culture vessel after the exposure of the bactericidal treatment medium is detached in the desired length of the planar fleece- or film-like material from the residual ribbon, wherein the ribbon with the respectively completely cultured part of the bacterially synthesized cellulose and the completely cultured bacterially synthesized cellulose-containing material, respectively, is removed from the culture vessel in a continuous or stepwise manner and is brought into contact with a removal device which substantially grips the ribbon from above and/or below in an initial region of the ribbon which is not intended for the utilization of the cellulose and the cellulose-containing material, respectively.

2. The method according to claim 1, **characterized in that** in the initial region of the ribbon one or more entraining elements are cultured into the bacterial cellulose and into the bacterial cellulose-containing material, respectively.

3. The method according to claim 2, **characterized in that** it is possible to touch the entraining elements by a removal device according to the principle of the hook-and-loop mount.

4. The method according to claim 1, **characterized in that** by the velocity with which the ribbon is removed from the culture vessel the thickness of the part of the ribbon in the culture vessel with the respectively completely cultured bacterially synthesized cellulose and with the completely cultured bacterially synthesized cellulose-containing material, respectively, is determined.

5. The method according to claim 1, **characterized in that** the ribbon with the respectively completely cultured part of the bacterially synthesized cellulose and the bacterially synthesized cellulose-containing material, respectively, for the purpose of treating with the bactericidal treatment medium after the removal from the culture vessel is guided through a preferably trough-like vessel in which the treatment medium is present.

6. The method according to claim 1, **characterized in that** the ribbon with the respectively completely cultured part of the bacterially synthesized cellulose and the bacterially synthesized cellulose-containing material, respectively, after the removal from the culture vessel and after the exposure of a bactericidal treatment medium is subjected to a purification treatment.

7. The method according to claim 6, **characterized in that** the purification treatment is realized by spraying a purifying liquid onto the ribbon one or more times.

8. The method according to claim 6, **characterized in that** the purification treatment is realized by immersing the ribbon into a purifying liquid.

9. The method according to claim 1, **characterized in that** during the culturing of the bacterially synthesized cellulose and the bacterially synthesized cellulose-containing material, respectively, in the culture vessel for the purpose of constant conditions of the culturing process respectively consumed components of the nutritional medium being inoculated with the bacterial culture are exchanged for respective new components.

10. The method according to claim 1, **characterized in that** during the culturing of the bacterially synthesized cellulose and the bacterially synthesized cellulose-containing material, respectively, in the culture vessel the temperature of the nutritional medium and/or the air is adjusted in a range of between 4 °C and 60 °C dependently on the cultured bacterial cellulose and/or the bacterial culture being used for the inoculation of the nutritional medium.

11. The method according to claim 1, **characterized in that** as bacterial culture for the inoculation of the nutritional medium a cellulose-producing bacterial strain is used.

## Revendications

1. Procédé de production de cellulose synthétisée par voie bactérienne et de matériau contenant de la cellulose et se présentant sous forme sensiblement bidimensionnelle, laquelle est cultivée dans un récipient de culture à l'interface d'un milieu nutritif, situé à l'intérieur et inoculé avec une culture bactérienne, par rapport à l'air, puis est retirée et, après retrait du récipient de culture, est exposée à l'action d'un milieu de traitement bactéricide, **caractérisé en ce que**
• la partie de la cellulose synthétisée par voie bactérienne ou du matériau contenant de la cellulose synthétisée par voie bactérienne, laquelle partie est entièrement cultivée à l'interface du milieu nutritif, inoculé avec la culture bactérienne, par rapport à l'air dans l'épaisseur définie, est retirée d'un récipient de culture en forme de cuve sans être séparée de la partie non encore entièrement cultivée de la cellulose ou du matériau contenant de la cellulose sous la forme d'une bande continue, sensiblement bidimensionnelle, en forme de nappe ou de film dans le plan de l'interface du milieu nutritif inoculé avec la culture bactérienne, la partie de la cellulose ou du matériau contenant de la cellulose, laquelle partie n'est pas encore entièrement cultivée à partir de cette bande, restant encore dans le récipient de culture, où sa culture se poursuit sans être dérangée par le retrait de la partie de la bande pourvue de la cellulose entièrement cultivée ou du matériau contenant de la cellulose et continuant à être retirée du récipient de culture dans la même bande, après avoir atteint l'épaisseur définie, sans être séparée de la partie déjà retirée de la bande de cellulose entièrement cultivée ou de matériau contenant de la cellulose entièrement cultivée et sans être séparée d'une autre partie, restant dans le récipient de culture, de la bande de cellulose non encore entièrement cultivée ou de matériau contenant de la cellulose non encore entièrement cultivée,
• la partie de la bande comprenant la cellulose synthétisée par voie bactérienne et entièrement cultivée ou le matériau contenant de la cellulose synthétisée par voie bactérienne et entièrement cultivée est exposée, après avoir été retirée du récipient de culture, au milieu de traitement bactéricide qui empêche un autre processus de culture de la cellulose ou du matériau contenant de la cellulose de cette partie de la bande,
• la partie de la bande, qui a été retirée du récipient de culture et qui comprend la cellulose synthétisée par voie bactérienne et entièrement cultivée ou le matériau contenant de la cellulose synthétisée par voie bactérienne et entièrement cultivée finie, est séparée du reste de la bande dans la longueur souhaitée du matériau sensiblement bidimensionnel en forme de nappe ou de film après l'action du milieu de traitement bactéricide, la bande qui comprend la partie entièrement cultivée de la cellulose synthétisée par voie bactérienne ou du matériau contenant de la cellulose synthétisée par voie bactérienne et entièrement cultivée étant retirée de manière continue ou discontinue du récipient de culture et étant amenée, dans une région initiale de la bande, non destinée à l'utilisation de la cellulose ou du matériau contenant de la cellulose, en contact avec un dispositif de retrait s'engageant avec la bande sensiblement en haut et/ou en bas.

2. Procédé selon la revendication 1, **caractérisé en ce que**, dans la région initiale de la bande, un ou plusieurs éléments d'entraînement sont introduits et cultivés dans la cellulose bactérienne ou dans le matériau contenant de la cellulose bactérienne.

3. Procédé selon la revendication 2, **caractérisé en ce que** les éléments d'entraînement peuvent être touchés par le biais d'un dispositif de retrait selon le principe de la fixation Velcro.

4. Procédé selon la revendication 1, **caractérisé en ce que** l'épaisseur de la partie de la bande dans le récipient de culture, qui comprend la cellulose synthétisée par voie bactérienne et entièrement cultivée ou le matériau contenant de la cellulose synthétisée par voie bactérienne et entièrement cultivée, est déterminée par la vitesse à laquelle la bande est retirée du récipient de culture.

5. Procédé selon la revendication 1, **caractérisé en ce que** la bande, qui comprend la partie entièrement cultivée de la cellulose synthétisée par voie bactérienne ou du matériau contenant de la cellulose synthétisée par voie bactérienne, est guidée, après retrait du récipient de culture, à travers un contenant de préférence en forme de cuve, dans lequel se trouve le milieu de traitement, en vue du traitement avec le milieu de traitement bactéricide.

6. Procédé selon la revendication 1, **caractérisé en ce que** la bande, qui comprend la partie entièrement cultivée de la cellulose synthétisée par voie bactérienne ou du matériau contenant de la cellulose synthétisée par voie bactérienne, est soumise à un traitement de nettoyage après retrait du récipient de culture et après action d'un milieu de traitement bactéricide.

7. Procédé selon la revendication 6, **caractérisé en ce que** le traitement de nettoyage est effectué par aspersion de la bande avec un liquide de nettoyage en une ou plusieurs fois.

8. Procédé selon la revendication 6, **caractérisé en ce que** le traitement de nettoyage est effectué par immersion de la bande dans un liquide de nettoyage.

9. Procédé selon la revendication 1, **caractérisé en ce que**, pendant la culture de la cellulose synthétisée par voie bactérienne ou du matériau contenant de la cellulose synthétisée par voie bactérienne dans le récipient de culture, des composants usés du milieu nutritif inoculé avec la culture bactérienne sont remplacés par de nouveaux composants appropriés afin de maintenir des conditions constantes du processus de culture.

10. Procédé selon la revendication 1, **caractérisé en ce que**, pendant la culture de la cellulose synthétisée par voie bactérienne ou du matériau contenant de la cellulose synthétisée par voie bactérienne dans le récipient de culture, la température du milieu nutritif et/ou de l'air dépend est réglée dans une plage comprise entre 4 °C et 60 °C en fonction de la culture de la cellulose bactérienne et/ou de la culture bactérienne utilisée pour l'inoculation du milieu nutritif.

11. Procédé selon la revendication 1, **caractérisé en ce qu'**une souche bactérienne productrice de cellulose est utilisée comme culture bactérienne pour inoculer le milieu nutritif.
